(19) Europäisches Patentamt
European Patent Office
Office européen des brevets

(11) Numéro de publication: **0 152 360**
**A2**

(12) **DEMANDE DE BREVET EUROPEEN**

(21) Numéro de dépôt: **85420018.5**

(22) Date de dépôt: **05.02.85**

(51) Int. Cl.⁴: **C 07 D 235/24**
**A 01 N 43/52**

(30) Priorité: **06.02.84 FR 8402118**

(43) Date de publication de la demande:
**21.08.85 Bulletin 85/34**

(84) Etats contractants désignés:
**AT BE CH DE FR GB IT LI LU NL SE**

(71) Demandeur: **RHONE-POULENC AGROCHIMIE**
**14-20, rue Pierre Baizet**
**F-69009 Lyon(FR)**

(72) Inventeur: **Giraudon, Raymond**
**5, rue des Colverts**
**F-77330 Lesigny(FR)**

(72) Inventeur: **Santini, Georges**
**92, rue Bugeaud**
**F-69006 Lyon(FR)**

(74) Mandataire: **Brachotte, Charles et al,**
**RHONE-POULENC AGROCHIMIE P.I.D. BP 9163**
**F-69263 Lyon Cedex 09(FR)**

(54) **Nouveaux dérivés du cyano-2 benzimidazole, leur préparation et leur utilisation comme fongicides.**

(57) L'invention concerne de nouveaux dérivés du cyano-2 benzimidazole.

Ces composés répondent à la formule (I):

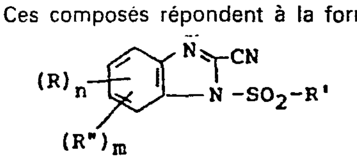

(II)

avec:

n 0, 1 ou 2,

m 1 ou 2,

R halogène; alkyle ou alkoxy ou alkylthio inférieurs éventuellement halogéné; $NO_2-$, $CN-$,

R' alkyle ou cycloalkyle inférieurs éventuellement halogènés, amino éventuellement substitués par un ou deux radicaux alkyles, ou un atome d'azote substitué par deux radicaux formant avec cet atome d'azote un hétérocycle comportant de 4 à 6 chaînons et de 1 à 3 hétéroatomes dans le cycle.

Ils sont utilisables en agriculture notamment pour la lutte contre les champignons phytopathogènes.

R" phénoxy éventuellement substitué ou phénylthio éventuellement substitué.

EP 0 152 360 A2

Croydon Printing Company Ltd

La présente demande de brevet concerne de nouveaux dérivés du cyano-2 benzimidazole ainsi que la préparation de ces composés. Elle concerne de plus les compositions pesticides, notamment antifongiques utilisables en agriculture et contenant comme matière active un de ces composés ainsi que les traitements pesticides et plus particulièrement les traitements antifongiques effectués au moyen de ces composés. Elle concerne enfin plusieurs composés chimiques utilisables pour la préparation des composés selon l'invention.

Les nouveaux dérivés du cyano-2 benzimidazole selon l'invention sont caractérisés en ce qu'ils répondent à la formule générale : (II)

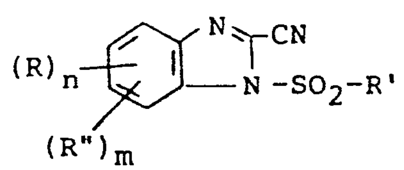

(II)

dans laquelle n, m, R, R' et R" ont les significations indiquées ci-après, étant entendu que, dans ce qui suit, sauf indication contraire, l'adjectif "inférieur" appliqué à un radical organique signifie que ce radical comporte au plus six atomes de carbone.

Dans la formule (II) :

n représente un nombre entier pouvant être égal à 0, 1 ou 2

m représente un nombre entier égal à 1 ou 2

R représente un atome d'halogène ou un radical alkyle inférieur éventuellement substitué par un ou plusieurs atomes d'halogène ; alkoxy inférieur éventuellement substitué par un ou plusieurs atomes d'halogène ; alkylthio inférieur éventuellement substitué par un ou plusieurs atomes d'halogène ; nitro ; cyano, étant entendu que, lorsque n est égal à 2, les substituants R peuvent être soit identiques, soit différents,

R' représente un radical alkyle ou cycloalkyle inférieur éventuellement substitué par un ou plusieurs atomes d'halogène (tel que, par exemple, les radicaux méthyle, éthyle, isopropyle, trichlorométhyle, trifluorométhyle, etc...) ; ou un radical amino éventuellement substitué par un ou deux radicaux alkyle inférieur, identiques ou différents, eux-mêmes éventuellement substitués (par exemple par un ou plusieurs halogènes) ; ou un atome d'azote substitué par deux radicaux formant avec cet atome d'azote un hétérocycle, lui-même éventuellement substitué( par exemple par un ou plusieurs substituants tels que atomes d'halogène ou groupes alkoxy inférieur ou alkylthio inférieur) comportant de 4 à 5 chainons et de 1 à 3 hétéroatomes dans le cycle (tel que par exemple le radical morpholino, le radical pyrrolidino, etc...).

R" représente un radical phénoxy éventuellement substitué ou phénylthio éventuellement substitué étant entendu que lorsque m est égal à 2, les substituants R" peuvent être soit identiques, soit différents.

Avantageusement le ou les substituants du radical phénoxy et/ou du radical phénylthio peuvent être choisis parmi les atomes d'halogène, de préférence fluor, chlore ou brome et les radicaux alkyle inférieur, cyano, nitro, alkoxy inférieur et halogénoalkyle inférieur, tel que par exemple radical trifluorométhyle.

Parmi les composés selon la formule (II), une sous-famille préférée du fait de ses remarquables propriétés antifongiques est constituée par les composés pour lesquels :

n est égal à 0 ou à 1 (de préférence n est égal à 0),

m est égal à 1,

R représente un atome d'halogène ou le radical cyano, nitro, trifluorométhyle, trifluorométhoxy ou trifluorométhylthio,

R' représente un radical dialkylamino ($C_2$-$C_4$) ou un radical alkyle ($C_1$-$C_3$) éventuellement halogéné(de préférence R' représente le radical diméthylamino),

R" représente un radical phénoxy substitué par de 1 à 3 substituants choisis parmi les atomes d'halogène et le radical trifluorométhyle.

L'invention concerne de plus un procédé pour préparer les composés selon la formule (II).

Ce procédé est caractérisé en ce qu'il consiste à faire réagir un cyano-2 benzimidazole de formule (III)

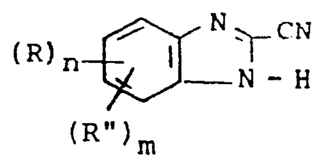

(III)

dans laquelle R, R", m et n ont le même signification que dans la formule (II), ou un sel (de préférence de métal alcalin ou d'ammonium) de ce cyano-2 benzimidazole (III), sur un halogénure de formule (IV)

$$X\text{-}SO_2R' \qquad (IV)$$

dans laquelle R' a la même signification que dans la formule (II) et X représente un atome d'halogène, de préférence un atome de chlore.

La réaction du cyano-2 benzimidazole (III) sur l'halogènure (IV) s'effectue avantageusement en présence d'un accepteur d'acide, en milieu anhydre ou non anhydre, dans un solvant, inerte dans les conditions de la réaction, généralement à la température d'ébullition du solvant. Comme accepteurs d'acide, on peut mentionner des bases minérales comme par exemple la soude ou la potasse, les carbonates de métaux alcalins ou de métaux alcalinoterreux, des bases azotées comme la triéthylamine. Comme solvants on utilise avantageusement des solvants aprotiques polaires, tels que par exemple, le diméthylformamide, le diméthylacétamide, le diméthylsulfoxyde, l'acétone, la méthyléthylcétone, l'acétonitrile, la N-méthylpyrrolidone.

Si désiré, cette réaction peut être effectuée en présence d'un catalyseur approprié. Comme catalyseur utilisable, on peut mentionner des catalyseurs de transfert de phases, tels que, par exemple, des dérivés d'ammonium quaternaire.

La réaction du sel (de métal alcalin ou d'ammonium) du cyano-2 benzimidazole (III) sur l'halogénure (IV) ne nécessite pas la présence d'un accepteur d'acide. Elle s'effectue en milieu anhydre ou non anhydre, dans un solvant, inerte dans les conditions de la réaction, généralement à la température d'ébullition du solvant. Les solvants aprotiques polaires cités plus haut peuvent être utilisés avantageusement dans cette réaction.

Si désiré, cette réaction peut être effectuée en présence d'un catalyseur approprié, tel que, par exemple, un catalyseur de transfert de phases (par exemple un dérivé d'ammonium quaternaire).

Le sel de métal alcalin ou d'ammonium du composé (III) est préparé dans une opération préalable, éventuellement effectuée in situ, par action d'une base appropriée (par exemple soude, potasse, ammoniaque) ou d'un carbonate de métal alcalin, ou d'un alcoolate de métal alcalin (par exemple méthylate de sodium ou éthylate de sodium ou potassium) sur ce composé (III).

En fin de réaction, quelque soit le procédé utilisé, le composé formé est isolé du milieu réactionnel par tout moyen en soi connu tel que, par exemple par distillation du solvant, ou par cristallisation du produit dans le milieu réactionnel, ou par filtration, puis, si nécessaire, ce composé est purifié par des méthodes usuelles telles que recristallisation dans un solvant approprié.

Le cyano-2 benzimidazole de formule (III), servant de produit de départ peut être préparé ci-après par action de l'ammoniaque sur un trihalogénométhyl-2 benzimidazole, de formule (V)

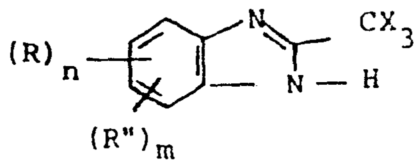

(V)

dans laquelle R, R", m et n ont la même signification que dans la formule (II) et X représente un atome d'halogène, de préférence le chlore, en opérant selon la méthode décrite dans la demande de brevet européen n° 0 087 375 (méthode A) et le brevet américain n° 3 576 818.

Il peut également être préparé selon l'une ou l'autre des méthodes B et C décrites dans la demande de brevet européen n° 0 087 375.

Le composé de formule (V) peut être préparé par action du trichloroacétimidate de méthyle, sur le diamino-1,2 benzène, de formule (VI)

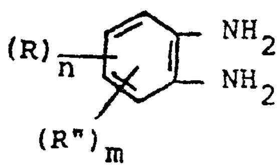

(VI)

dans laquelle R, R", m et n ont la même signification que dans la formule (V), ou sur un sel de ce composé formé avec un acide fort.

La réaction s'effectue avantageusement dès la température ordinaire en milieu acide acétique, ou dans des alcanols inférieurs tels que l'éthanol ou le méthanol. Elle peut également être effectuée à la température de reflux des solvants utilisés. Si désiré le passage du diamino-1,2 benzène de formule (VI) au cyano-2 benzimidazole (III) peut s'effectuer en effectuant successivement les deux étapes décrites plus haut sans isoler le trihalogénométhyl-2 benzimidazole de formule (V) formé intermédiairement.

Le diamino-1,2 benzène de formule (VI) peut être préparé à partir de la nitroaniline de formule (VII)

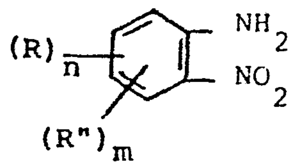

(VII)

dans laquelle R, R", m et n ont la même signification que dans la formule (V), par réduction du groupe nitro en groupe amino. Cette réduction peut s'effectuer par tout moyen en soi connu pour permettre la réduction d'un nitrobenzène en aniline, sans effectuer les autres substituants, présents sur le noyau benzénique par exemple en faisant agir le chlorure stanneux en présence d'acide chlorhydrique concentré.

La nitroaniline (VII) peut être obtenue par action de l'acide sulfurique sur la N-(nitrophényl)-acétamide correspondante.

Les composés répondant aux formules (III), (V) et (VI) et (VII) sont compris dans le cadre de la présente invention, en tant que produits nouveaux utilisables pour la mise en oeuvre du procédé de préparation décrit plus haut.

Les exemples ci-après, décrits à titre non limitatif, illustrent la préparation des composés selon l'invention et leur utilisation comme fongicides. Les structures des composés décrits dans ces exemples ont été confirmées par spectrométrie de résonance magnétique nucléaire (RMN) et/ou par spectrométrie infrarouge.

Exemple 1

Préparation du cyano-2(chloro-2 trifluorométhyl-4 phénoxy)-5 diméthylsulfamoyl-1 benzimidazole (composé 1A) en mélange avec le cyano-2(chloro-2 trifluorométhyl-4 phénoxy)-6 diméthylsulfamoyl-1 benzimidazole (composé 1B).

A une suspension de 1,04 g (16,2 millimoles) de potasse en écailles dans 60 ml d'acétone, on ajoute, à température ambiante (20°C environ) 5,5 g (16,2 millimoles)

de cyano-2(chloro-2 trifluorométhyl-4 phénoxy)-5 benzimidazole. On observe alors la dissolution complète des réactifs et une élévation de la température qui atteint 35°C.

On ajoute alors au mélange réactionnel, en 10 minutes, 1,75 ml de chlorure de diméthylsulfamoyle (16,2 millimoles) et le mélange réactionnel est agité pendant 18 heures puis concentré. On obtient une huile qui est chromatographiée sur 200 g de silice, avec comme éluant le chlorure de méthylène.

On obtient ainsi 4,2 g du composé désiré, sous la forme d'une huile qui cristallise au bout d'une semaine. Point de fusion 90°C. Rendement 58,3 %.

Le cyano-2(chloro-2 trifluorométhyl-4 phénoxy)-5 benzimidazole a été préparé à partir du (chloro-2 trifluorométhyl-4 phénoxy)-4 diamino-1,2 benzène comme indiqué ci-après :

A une solution de 35 g (0,122 mole) de (chloro-2 trifluorométhyl-4 phénoxy)-4 diamino-1,2 benzène dans 165 ml d'acide acétique, on ajoute 15,1 ml de trichloroacétimidate de méthyle (0,122 mole). On observe au bout de 15 minutes une élévation de la température jusqu'à 52°C. On laisse alors refroidir le mélange à 25°C, en le maintenant sous agitation pendant 18 heures.

La solution ainsi obtenue est alors coulée goutte à goutte, en 20 minutes, sur de l'ammoniaque (2 litres) à 84 %, à une température comprise entre 10 et 15°C. Le mélange réactionnel est maintenu sous agitation à 25°C pendant 3 heures puis filtré. Le solide obtenu est chromatographié sur colonne de silice avec comme éluant un mélange 80/20 de toluène et d'acétate d'éthyle. On obtient 6,8 de cyano-2(chloro-2 trifluorométhyl-4 phénoxy)-5 benzimidazole fondant à 192°C. Rendement 16,4 %.

Le (chloro-2 trifluorométhyl-4 phénoxy)-4 diamino-1,2 benzène a été préparé comme indiqué ci-après :

On ajoute par petites fractions 44 g (0,138 mole) de amino-1 nitro-2 (chloro-2 trifluorométhyl-4 phénoxy)-5 benzène à une solution de 117,5 g (0,52 mole de dihydrate de chlorure stanneux, dans 600 ml d'acide chlorhydrique concentré (densité 1,19). L'addition conduite en 15 minutes, provoque une élévation de température de 20 à 45°C.

On agite ensuite le mélange réactionnel pendant 2 heures à) 25°C. Après avoir ajouté un litre d'eau distillée et un litre de chlorure de méthylène, on alcalinise jusqu'à pH 13, à l'aide de soude concentrée, en refroidissant, afin de ne pas dépasser 25°C. Après décantation, la phase aqueuse est de nouveau extraite par deux fois 400 ml de chlorure de méthylène. Les phases organiques rassemblées sont séchées sur sulfate de sodium, puis concentrées sous vide pour conduite à 35 g rendement 88,5 % de diamino-1,2 (chloro-2 trifluorométhyl-4 phénoxy)-4 benzène, sous la forme d'une huile brune.

Le amino-1 nitro-2 (chloro-2 trifluorométhyl-4 phénoxy)-5 benzène a été préparé comme indiqué ci-après :

A 350 ml d'acide sulfurique concentré, on ajoute par petites fractions en vingt minutes 75,5 g (0,2 mole) de N-[nitro-2 (chloro-2 trifluorométhyl-4 phénoxy)-5 phényl] acétamide. La température monte à 30°C. Le mélange réactionnel est ensuite chauffé à 50°C pendant deux heures puis après refroidissement versé sur 1 kg de glace-eau. Le solide ainsi obtenu est purifié par chromatographie pour donner 44 g de amino-1 nitro-2 (chloro-2 trifluorométhyl-4 phénoxy)-5 benzène fondant à 100°C. Rendement 69 %.

Exemple 2

En opérant selon la méthode décrite dans l'exemple précédent, les composés ou mélanges de composés ci-après ont été préparés :

2A + 2B : mélange 50/50 de cyano-2 diméthylsulfamoyl-1 phénylthio-5 benzimidazole et de cyano-2

diméthylsulfamoyl-1 phénylthio-6 benzimidazole. Point de fusion : 100°C.

3A + 3B : mélange 93/7 ou 7/93 de cyano-2 diméthylsulfamoyl-1 phénoxy-6 benzimidazole et de cyano-2 diméthylsulfamoyl-1 phénoxy-5 benzimidazole. Point de fusion 170°C.

4A + 4B : mélange 66/34 ou 34/66 de cyano-2 diméthylsulfamoyl-1 phénoxy-6 trifluorométhyl-5 benzimidazole et de cyano-2 diméthylsulfamoyl-1 phénoxy-5 trifluorométhyl-6 benzimidazole. Point de fusion : 138°C. Rendement 69,4 %

5A + 5B : mélange 60/40 ou 40/60 de cyano-2 diméthylsulfamoyl-1(dichloro-2,4 phénoxy)-6 trifluorométhyl-5 benzimidazole et de cyano-2 trifluorométhyl-6 diméthylsulfamoyl-1(dichloro-2,4 phénoxy)-5 benzimidazole fondant à 115-125°C. Rendement 81 %.

Les formules de ces composés sont indiquées dans la table I, à la fin de la description.

Exemple 3

Test en serre sur mildiou de la tomate

Des plants de tomate (*Lycopersicum esculentum*), de variété Marmande, sont cultivés dans des godets. Lorsque ces plants sont agés d'un mois (stade 5 à 6 feuilles, hauteur 12 à 15 cm), ils sont traités par pulvérisation au moyen d'une suspension ou solution aqueuse de la matière à tester, à la concentration désirée et contenant 0,02% d'un condensat de monoléate de sorbitan et de 20 molécules d'oxyde d'éthylène. Chaque plant de tomate reçoit environ 5ml de la solution ou dispersion. Pour chaque concentration de matière active à tester, le traitement est effectué sur huit plants. Des plants utilisés comme témoins sont traités

par une solution ne contenant pas de matière active, mais contenant 0,02% du même condensat de monooléate de sorbitan et d'oxyde d'éthylène.

Après séchage pendant 4 heures, on contamine chaque plant par pulvérisation au moyen d'une suspension aqueuse de spores de <u>Phytophthora infestans</u>, responsable du mildiou de la tomate, à raison d'environ 1 ml/plant (soit environ $2.10^5$ spores par plant).

Après cette contamination, les plants de tomate sont mis en incubation pendant deux à trois jours à 17°C environ en atmosphère saturée d'humidité, puis pendant quatre jours à 22°C environ sous 70% à 80% d'humidité relative.

Sept jours après la contamination, on compare les résultats obtenus dans le cas des plants traités par la matière active à tester à ceux obtenus dans le cas des plants utilisés comme témoins et on détermine la concentration minimale inhibitrice entraînant de 95 à 100% d'inhibition du développement du champignon considéré (CMI 95-100).

Dans ces conditions, on observe que, pour les composés ou mélanges de composés décrits dans les exemples précédents, cette concentration a été respectivement la suivante :

| Composé ou mélange testé | CMI (95-100) Phytophthora infestans en mg/l |
|---|---|
| 1A + 1B | inférieure à 125 |
| 2A + 2B | supérieure à 500 |
| 3A + 3B | supérieure à 500 |
| 4A + 4B | inférieure à 500 |
| 5A + 5B | inférieure à 125 |

Exemple 4

Test en serre sur mildiou du tabac

On opère comme à l'exemple précédent si ce n'est que les végétaux sont des plants de tabac (Nicotiana tabacum) de variété Samson et que ces plants sont contaminés par des spores de Peronospora tabacina, responsable du mildiou du tabac.

Dans ces conditions, on a observé que, pour les composés ou mélanges de composés décrits dans les exemoles précédents, les concentrations minimales inhibitrices entraînant de 95 à 100% d'inhibition du champignon considéré (CMI 95-100) sont respectivement les suivantes :

| Composés ou mélange testés | CMI (95-100) Peronospora tabacina en mg/l |
|---|---|
| 1A + 1B........... | inférieure à 8 |
| 2A + 2B........... | supérieure à 500 |
| 3A + 3B........... | supérieure à 500 |
| 4A + 4B........... | supérieure à 500 |
| 5A + 5B........... | 500 |

Les composés selon l'invention sont avantageusement utilisés comme antifongiques dans le domaine agricole. Ils présentent une action par contact et par systèmie et peuvent être employés de préférence à tire préventif mais aussi à titre curatif pour la lutte contre divers champignons phytopathogènes tels que par exemple de nombreux phycomycètes et basidiomycètes, notamment Phytophthora infestans (mildiou de la pomme de terre, du tabac, de la tomate) et Plasmopara viticola (mildiou de la vigne). Certains de ces composés peuvent être également utilisés avantageusement comme antiacariens notamment vis à vis des acariens phytophages.

Pour leur emploi dans la pratique, les composés selon l'invention ne sont généralement pas utilisés seuls. Le plus souvent ils sont utilisés dans des compositions qui comprennent en général, en plus de la matière active, un support (ou diluant) inerte et/ou un agent tensioactif compatibles avec la matière active.

Ces compositions font également partie de la présente invention. Elles contiennent habituellement de 0,001 à 95% en poids de matière active. Leur teneur en agent tensioactif est en général comprise entre 0% et 20% en poids.

Par le terme "support", dans le présent exposé, on désigne une matière organique ou minérale, naturelle ou synthétique, avec laquelle la matière active est associée pour faciliter son application sur la plante, sur des graines ou sur le sol. Ce support est donc généralement inerte et il doit être acceptable en agriculture, notamment par la plante traitée. Le support peut être solide (argiles, silicates naturels ou synthétiques, silice, craies, résines, cires, engrais solides, etc...) ou liquide (eau, alcools, cétones, fractions de pétrole, hydrocarbures aromatiques ou paraffiniques, hydrocarbures chlorés, gaz liquéfiés, etc...).

L'agent tensioactif peut être un agent émulsionnant, dispersant ou mouillant de type ionique ou non ionique. On peut citer par exemple des sels d'acides polyacryliques ; des sels d'acides lignosulfoniques des sels d'acides phénolsulfoniques ou naphtalènesulfoniques ; des polycondensats d'oxyde d'éthylène sur des alcools gras ou sur des acides gras ou sur des amines grasses ou sur des phénols substitués (notamment des alkylphénols ou des arylphénols ou le styrylphénol) ; des sels d'esters d'acides sulfosucciniques ; des dérivés de la taurine (notamment des alkyltaurates) ; des esters phosphoriques d'alcools ou de phénols polyoxyéthylés. La présence d'au moins un agent tensioactif est généralement indispensable

surtout lorsque le support inerte n'est pas soluble dans l'eau, et que l'agent vecteur de l'application est l'eau.

Les compositions utilisées dans l'invention peuvent être sous des formes assez diverses, solides ou liquides.

Comme formes de compositions solides, on peut citer les poudres pour poudrage (à teneur en matières actives pouvant aller jusqu'à 100 %).

Comme formes de compositions liquides ou destinées constituer des compositions liquides lors de l'application, on peut citer les solutions, en particulier les concentrés solubles dans l'eau, les concentrés émulsionnables, les émulsions, les suspensions concentrées, les aérosols, les poudres mouillables (ou poudre à pulvériser), les granulés, les pâtes.

Les concentrés émulsionnables ou solubles comprennent le plus souvent 10 à 80 % de matière active, les émulsions ou solutions prêtes à l'application contenant, quant à elles, 0,001 à 20 % de matière active. En plus de la matière active et du solvant, les concentrés émulsionnables peuvent contenir, quand c'est nécessaire, un co-solvant approprié et de 2 à 20 % d'additifs appropriés, comme des stabilisants, des agents tensioactifs, notamment des émulsifs, des agents de pénétration, des inhibiteurs de corrosion, des colorants, des adhésifs.

A partir de ces concentrés, on peut obtenir par dilution avec de l'eau des émulsions de toute concentration désirée, qui conviennent particulièrement à l'application sur les cultures.

Les suspensions concentrées, également applicables en pulvérisation, sont préparées de manière à obtenir un produit fluide stable ne se déposant pas et elles contiennent habituellement de 10 à 75 % de matière active, de 0,5 à 15 % d'agents tensioactifs, de 0,1 à 10 % d'agents thixotropes, de 0 à 10 % d'additifs appropriés, comme des anti-mousses, des inhibiteurs de corrosion, des stabilisants, des agents de pénétration et des adhésifs et,

comme support, de l'eau ou un liquide organique dans lequel la matière active est peu ou pas soluble : certaines matières solides organiques ou des sels minéraux peuvent être dissous dans le support pour aider à empêcher la sédimentation ou comme antigels pour l'eau.

A titre d'exemple, voici la composition de plusieurs suspensions aqueuses selon l'invention :

Exemple A :

On prépare une suspension aqueuse comprenant :

- matière active (composé n° 1A + 1B) .........500 g/l
- agent mouillant (alcool synthétique en $C_{13}$ polyéthoxylé) .............................. 10 g/l
- agent dispersant (phosphate de polyaryl phénol éthoxylé salifié) ......................... 50 g/l
- antigel (propylèneglycol) .................. 100 g/l
- épaississant (polysacharide) ............... 1,6 g/l
- biocide (méthylhydroxy-4 benzoate sodé) .... 3,3 g/l
- eau ................................Q.S.P.1 litre.

On obtient ainsi une suspension concentrée fluide de couleur crème.

Exemple B - Suspension aqueuse :

On prépare une suspension aqueuse comprenant :

- matière active (composé n° 1A + 1B)......... 100 g/l
- agent mouillant (alkylphénol polyéthoxylé .. 5 g/l
- agent dispersant (Naphtalène sulfonate de Na 10 g/l
- antigel (Propylèneglycol) .................. 100 g/l
- épaississant (Polysacharide) ............... 3 g/l
- biocide (Formaldéhyde) ..................... 1 g/l
- eau ................................Q.S.P.1 litre.

Exemple C - Suspension aqueuse :

On prépare une suspension aqueuse comprenant :

- matière active (composé n° 1A + 1B)......... 250 g/l
- agent mouillant (alcool synthétique en $C_{13}$ polyéthoxylé .............................. 10 g/l
- agent dispersant (lignosulfonate de solium) 15 g/l
- antigel (urée) ............................. 50 g/l

- épaississant (Polysacharide) .............. 2,5 g/l
- biocide (Formaldéhyde) .................... 1 g/l
- eau ...............................Q.S.P.1 litre.

Les poudres mouillables (ou poudre à pulvériser) sont habituellement préparées de manière qu'elles contiennent 20 à 95 % de matière active, et elles contiennent habituellement, en plus du support solide, de 0 à 5 % d'un agent mouillant, de 3 à 10 % d'un agent dispersant, et, quand c'est nécessaire, de 0 à 10 % d'un ou plusieurs stabilisants et/ou autres additifs, comme des agents de pénétration, des adhésifs, ou des agents antimottants, colorants, etc...

A titre d'exemple, voici la composition de plusieurs poudres mouillables.

- matière active (composé n° 1A + 1B) 50 %
- alcool gras éthoxylé (agent mouillant)......... 2,5 %
- styrylphénol éthoxylé (agent dispersant)....... 5 %
- craie (support inerte) ........................42,5 %

Exemple E : Poudre mouillable à 10 %

- matière active (composé n° 1A + 1B°.............. 10 %
- alcool synthétique oxo de type ramifié, en $C_{13}$ éthoxylé par 8 à 10 oxyde d'éthylène (agent mouillant) ..................................0,75 %
- lignosulfonate de calcium neutre (agent dispersant ......................................... 12 %
- carbonate de calcium (charge inerte) .......qsp 100 %

Exemple F : Poudre mouillable à 75 % contenant les mêmes ingrédients que dans l'exemple précédent, dans les proportions ci-après :

- matière active .............................. 75 %
- agent mouillant ............................1,50 %
- agent dispersant ..............................8 %
- carbonate de calcium (charge inerte) .......qsp 100 %

Exemple G : Poudre mouillage à 90 %

- matière active (composé n° 1 selon l'invention ..90 %
- alcool gras éthoxylé (agent mouillant ........... 4 %

- styrylphénol éthoxylé (agent dispersant) ........ 6 %

Exemple H : Poudre mouillable à 50 %

- matière active (composé n° 1 selon l'invention ..50 %
- mélange de tensio-actifs anioniques et non ioniques (agent mouillant) ......................2,5 %
- lignosulfonate de sodium neutre (agent dispersant) ........................................ 5 %
- argile kaolinique (support inerte) .............42,5 %

Pour obtenir ces poudres à pulvériser ou poudres mouillables, on mélange intimement la matière active dans des mélangeurs appropriés avec les substances additionnelles et on broie avec des moulins ou autres broyeurs appropriés. On obtient par là des poudres à pulvériser dont la mouillabilité et la mise en suspension sont avantageuses ; on peut les mettre en suspension avec de l'eau à toute concentration désirée et cette suspension est utilisable très avantageusement en particulier pour l'application sur les feuilles des végétaux.

Les composés selon l'invention sont avantageusement formulés sous la forme de granulés dispersibles dans l'eau également compris dans le cadre de l'invention.

Ces granulés dispersibles, de densité apparente généralement comprise entre environ 0,3 et 0,6, ont une dimension de particules généralement comprise entre environ 150 et 2000 et de préférence entre 300 et 1500 microns.

La teneur en matière active (composé n° 1A + 1B) de ces granulés est généralement comprise entre environ 1 % et 90 %, et de préférence entre 25 % et 90 %.

Le reste du granulé est essentiellement composé d'une charge solide et éventuellement d'adjuvants tensio-actifs conférant au granulé des propriétés de dispersibilité dans l'eau. Ces granulés peuvent être essentiellement de deux types distincts selon que la charge retenue est soluble ou non dans l'eau. Lorsque la charge est hydrosoluble, elle peut être minérale et de préférence organique. On a obtenu d'excellents résultats avec l'urée.

Dans le cas d'une charge insoluble, celle-ci est de préférence minérale, comme par exemple le kaolin ou la bentonite. Elle est alors accompagnée d'agents tensio-actifs (à raison de 2 à 20 % en poids du granulé), adjuvants tensio-actifs dont plus de la moitié est constituée par au moins un agent dispersant, essentiellement anionique, tel qu'un poly(naphtalène sulfonate alcalin ou alcalino terreux) ou un lignosulfonate alcalin ou alcalino-terreux, le reste étant constitué par des mouillants non ioniques ou anioniques tel qu'un alcoyl naphtalène sulfonate alcalin ou alcalino-terreux.

Par ailleurs, bien que cela ne soit pas indispensable, on peut ajouter d'autres adjuvants tels que des agents anti-mousse.

Le granulé selon l'invention peut être préparé par mélange des ingrédients nécessaires puis granulation selon plusieurs techniques en soi connues (drageoir, lit fluide, atomiseur, extrusion, etc..). On termine généralement par un concassage suivi d'un tamisage à la dimension de particule choisie dans les limites mentionnées ci-dessus.

De préférence, il est obtenu par extrusion, en opérant comme indiqué dans les exemples ci-après.

Exemple I : Granulés dispersibles à 90 % de matière active

Dans un mélangeur, on mélange 90 % en poids de matière active (composé n° 1) et 10 % d'urée en perles. Le mélange est ensuite broyé dans un broyeur à broches. On obtient une poudre que l'on humidifie avec environ 8 % en poids d'eau. La poudre humide est extrudée dans une extrudeuse à rouleau perforé. On obtient un granulé qui est séché, puis concassé et tamisé, de façon à ne garder respectivement que les granulés d'une dimension comprise entre 150 et 2000 microns.

Exemple J : Granulés dispersibles à 75 % de matière active

Dans un mélangeur, on mélange les constituants suivants :

- matière active (composé n° 1) .................. 75 %
- agent mouillant (alkylnaphtalène sulfonate de sodium ............................................ 2 %
- agent dispersant (polynaphtalène sulfonate de sodium) ............................................ 8 %
- charge inerte insoluble dans l'eau (kaolin) ..... 15 %

Ce mélange est granulé en lit fluide, en présence d'eau, puis séché, concassé et tamisé de manière à obtenir des granulés de dimension comprise entre 0,16 et 0,40 mm.

Ces granulés peuvent être utilisés seuls, en solution ou dispersion dans de l'eau de manière à obtenir la dose cherchée. Ils peuvent aussi être utilisés pour préparer des associations avec d'autres matières actives, notamment fongicides, ces dernières étant sous la forme de poudres mouillables, ou de granulés ou suspensions aqueuses.

Comme cela a déjà été dit, les dispersions et émulsions aqueuses, par exemple des compositions obtenues en diluant à l'aide d'eau une poudre mouillable ou un concentré émulsionnable selon l'invention, sont comprises dans le cadre général des compositions utilisables dans la présente invention. Les émulsions peuvent être du type eau-dans-l'huile ou huile-dans-l'eau et elles peuvent avoir une consistance épaisse comme celle d'une "mayonnaise".

L'invention concerne de plus un procédé de traitement des végétaux contre les champignons phytopathogènes.

Ce procédé est caractérisé en ce qu'il consiste à appliquer sur ces végétaux une quantité efficace d'une composition contenant comme matière active un composé selon la formule (II). Par "quantité efficace" on entend une quantité suffisante pour permettre le contrôle et la destruction des champignons présents sur ces végétaux. Les doses d'utilisation peuvent toutefois varier dans de larges limites selon le champignon à combattre, le type de culture, les conditions climatiques, et selon le composé utilisé.

En pratique des doses allant de 5 g/hl à 100 g/hl correspondant sensiblement à des doses de matière active par hectare de 50 g/ha à 1000 g/ha environ donnent généralement de bons résultats.

TABLE 1

| COMPOSE No. | FORMULE | POINT DE FUSION |
|---|---|---|
| 1A | 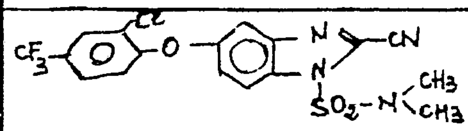 | 192°C |
| 1B | 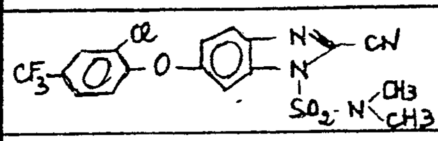 | |
| 2A | 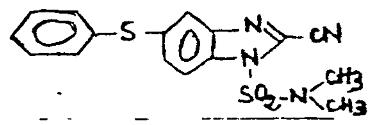 | Mélange 2A + 2B (50/50) : 100°C |
| 2B | 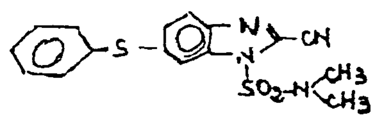 | |
| 3A | 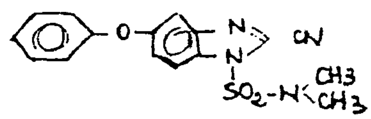 | 94°C |
| 3B | 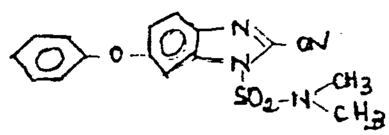 | 170°C |
| 4A | 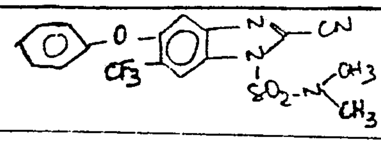 | Mélange 4A + 4B (66/34 ou 34/66) : 138°C |
| 4B | 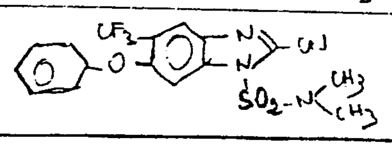 | |
| 5A | 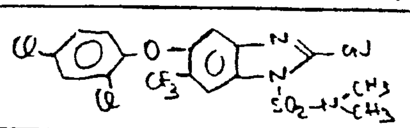 | Mélange 5A + 5B (60/40 ou 40/60) : 115-125°C |
| 5B | 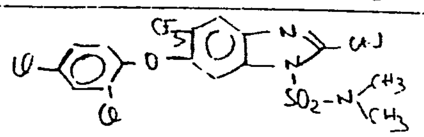 | |

REVENDICATIONS POUR ETATS CONTRACTANTS : BE, CH, DE, FR, GB, IT, Li, LU, NL, SE

1) Dérivé du cyano-2 benzimidazole caractérisé en ce qu'il répond à la formule générale :

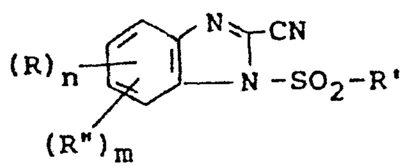

dans laquelle :

n représente un nombre entier égal à 0, 1 ou 2,

m représente un nombre entier égal à 1 ou 2,

R représente un atome d'halogène ou un radical alkyle inférieur, éventuellement substitué par un ou plusieurs atomes d'halogènes ; alkoxy inférieur éventuellement substitué par un ou plusieurs atomes d'halogènes ; alkylthio inférieur éventuellement substitué par un ou plusieurs atomes d'halogènes ; nitro ; cyano, étant entendu que, lorsque n est égal à 2, les substituants R peuvent être soit identiques soit différents ;

R' représente un radical alkyle ou cycloalkyle inférieur, éventuellement substitué par un ou plusieurs atomes d'halogènes ; ou un radical amino éventuellement substitué par un ou deux radicaux alkyle inférieur, identiques ou différents, eux-mêmes éventuellement substitués ; ou un atome d'azote substitué par deux radicaux formant avec cet atome d'azote un hétérocycle, lui-même éventuellement substitué, comportant de 4 à 6 chaînons et de 1 à 3 hétéroatomes dans le cycle ;

R" représente un radical phénoxy éventuellement substitué ou phénylthio éventuellement substitué

2) Composé selon la revendication 1 caractérisé en ce que :

n est égal à 0 ou à 1,

m est égal à 1,

R représente un atome d'halogène ou un radical cyano, nitro, trifluorométhyle, trifluorométhoxy ou trifluorométhylthio,

R' représente un radical dialkylamino ($C_2$-$C_4$) ou un radical alkyle ($C_1$-$C_3$) éventuellement halogéné,

R" représente un radical phénoxy substitué par 1 à 3 substituants choisis parmi les atomes d'halogène et le radical trifluorométhyle.

3) Composé selon la revendication 2, caractérisé en ce que :

n est égal à 0,

m est égal à un,

R' représente le radical diméthylamino et

R" représente un radical phénoxy substitué par de un à deux substituants choisis parmi les atomes d'halogène et le radical trifluorométhyle.

4) Composition pesticide, à usage agricole, utilisable notamment pour la lutte contre les champignons phytopathogènes, caractérisée en ce qu'elle comprend comme matière active un composé selon l'une des revendication 1 à 3.

5) Composition selon la revendication 4 caractérisée en ce que, en plus de la matière active, elle comprend un support inerte et/ou un agent tensioactif utilisables en agriculture.

6) Composition selon la revendication 5 caractérisée en ce qu'elle comprend de 0,001 % à 95 % en poids de matière active et de 0 à 20 % en poids d'agent tensioactif.

7) Procédé de préparation d'un composé selon la revendication 1) caractérisé en ce qu'il consiste à faire réagir un cyano-2 benzimidazole de formule :

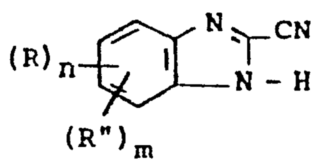

dans laquelle n, m, R et R" ont la même signification que dans la revendication 1), ou un sel de ce cyano-2 benzimidazole, sur un halogénure de formule :

$$X-SO_2R'$$

dans laquelle R' a la même signification que dans la revendication 1) et X représente un atome d'halogène.

8) Procédé selon la revendication 7) caractérisé en ce que la réaction entre le cyano-2 benzimidazole et l'halogénure s'effectue dans un solvant aprotique polaire, en présence d'un accepteur d'acide.

9) Procédé selon l'une des revendications 7) ou 8) caractérisé en ce que la réaction entre le sel de métal alcalin ou d'ammonium du cyano-2 benzimidazole et l'halogénure s'effectue dans un solvant aprotique polaire.

10) Procédé de traitement des végètaux contre les champignons phytopathogènes caractérisé en ce qu'il consiste à appliquer sur ces végétaux une quantité efficace d'un composé selon l'une des revendications 1) à 3).

11) Composé répondant à l'une ou l'autre des formules ci-après :

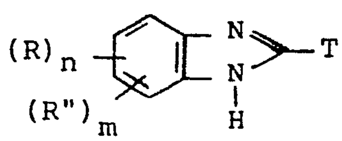

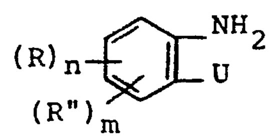

dans lesquelles m, n, R et R", ont la même signification que dans la revendication 1), T représente le radical cyano ou un radical trihalogénométhyle et U représente le radical amino ou nitro.

REVENDICATIONS POUR L'ETAT CONTRACTANT : AT

1) Composition pesticide, à usage agricole, utilisable notamment pour la lutte contre les champignons phytopathogènes, caractérisée en ce qu'elle comprend comme matière active un composé répondant à la formule générale :

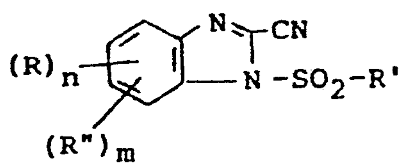

dans laquelle :

n représente un nombre entier égal à 0, 1 ou 2,

m représente un nombre entier égal à 1 ou 2,

R représente un atome d'halogène ou un radical alkyle inférieur, éventuellement substitué par un ou plusieurs atomes d'halogènes ; alkoxy inférieur éventuellement substitué par un ou plusieurs atomes d'halogènes ; alkylthio inférieur éventuellement substitué par un ou plusieurs atomes d'halogènes ; nitro ; cyano, étant entendu que, lorsque n est égal à 2, les substituants R peuvent être soit identiques soit différents ;

R' représente un radical alkyle ou cycloalkyle inférieur, éventuellement substitué par un ou plusieurs atomes d'halogènes ; ou un radical amino éventuellement substitué par un ou deux radicaux alkyle inférieur, identiques ou différents, eux-mêmes éventuellement substitués ; ou un atome d'azote substitué par deux radicaux formant avec cet atome d'azote un hétérocycle, lui-même éventuellement substitué, comportant de 4 à 6 chaînons et de 1 à 3 hétéroatomes dans le cycle ;

R" représente un radical phénoxy éventuellement substitué

2) Composition selon la revendication 1 caractérisée en ce que :

n est égal à 0 ou à 1,

m est égal à 1,

R représente un atome d'halogène ou un radical cyano, nitro, trifluorométhyle, trifluorométhoxy ou trifluorométhylthio,

R' représente un radical dialkylamino ($C_2$-$C_4$) ou un radical alkyle ($C_1$-$C_3$) éventuellement halogéné,

R" représente un radical phénoxy substitué par 1 à 3 substituants choisis parmi les atomes d'halogène et le radical trifluorométhyle.

3) Composition selon la revendication 2, caractérisée en ce que :

n est égal à 0,

m est égal à un,

R' représente le radical diméthylamino et

R" représente un radical phénoxy substitué par de un à deux substituants choisis parmi les atomes d'halogène et le radical trifluorométhyle.

4) Composition selon l'une des revendications 1 à 3, caractérisée en ce que, en plus de la matière active, elle comprend un support inerte et/ou un agent tensioactif utilisables en agriculture.

5) Composition selon la revendication 4 caractérisée en ce qu'elle comprend de 0,001 % à 95 % en poids de matière active et de 0 à 20 % en poids d'agent tensioactif.

6) Procédé de préparation d'un composé de formule :

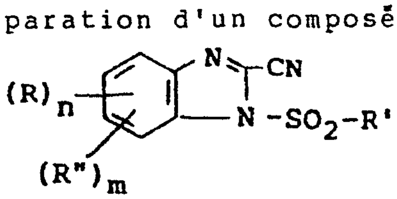

dans laquelle n, m, R, R' et R" ont la même signification que dans la revendication 1, cacactérisé en ce qu'il consiste à faire réagir un cyano-2 benzimidazole de formule :

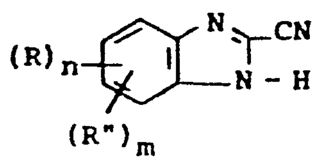

dans laquelle n, m, R et R" ont la même signification que précédemment, ou un sel de ce cyano-2 benzimidazole, sur un halogénure de formule :

$$X-SO_2R'$$

dans laquelle R' a la même signification que précédemment et X représente un atome d'halogène.

7) Procédé selon la revendication 6) caractérisé en ce que la réaction entre le cyano-2 benzimidazole et l'halogénure s'effectue dans un solvant aprotique polaire, en présence d'un accepteur d'acide.

8) Procédé selon la revendication 6) caractérisé en ce que la réaction entre le sel de métal alcalin ou d'ammonium du cyano-2 benzimidazole et l'halogénure s'effectue dans un solvant aprotique polaire.

9) Procédé de traitement des végétaux contre les champignons phytopathogènes caractérisé en ce qu'il consiste à appliquer sur ces végétaux une quantité efficace d'une composition selon l'une des revendications 1) à 5).